Europäisches Patentamt

European Patent Office

Office européen des brevets

(19)

(11) **EP 1 138 780 A1**

(12) **EUROPEAN PATENT APPLICATION**

(43) Date of publication:
**04.10.2001 Bulletin 2001/40**

(51) Int Cl.[7]: **C12Q 1/68**

(21) Application number: **00107036.6**

(22) Date of filing: **31.03.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Roche Diagnostics GmbH**
**68305 Mannheim (DE)**

(72) Inventors:
• **Tabiti, Karim, Dr.**
**82343 Pöcking (DE)**

• **Sagner, Gregor, Dr.**
**82377 Penzberg (DE)**
• **Gutekunst, Martin, Dr.**
**82390 Eberfing (DE)**
• **Soong, Richie, Dr.**
**82327 Tutzing (DE)**

(74) Representative: **Schreiner, Siegfried, Dr. et al**
**Roche Diagnostics GmbH,**
**Patent Department Pharma (TR-E),**
**P.O. Box 1152**
**82372 Penzberg (DE)**

(54) **Detection of disseminated tumor cells by quantitative RT-PCR of cytokeratin 20 mRNA**

(57)    A method for the detection of cytokeratin 20 (CK-20) mRNA in the tissue or body fluid sample of a patient, which is characterized by i) isolating RNA from said sample; ii) preparing cDNA from said RNA by reverse transcription and dividing said cDNA into two compartments; iii) performing polymerase chain reaction (PCR) with CK-20 specific primers in the first compartiment; iv) performing PCR with primers specific for a housekeeping gene in the second compartment; v) calculating the relative ratio of mRNA of CK-20 to the housekeeping gene from the results of steps iii) and iv) as means for amount of the CK-20 mRNA in the sample, provides a sensitive and quantitative method for the detection of CK-20.

| | Colorectal | Breast | H&NSCC | Melanoma | Vulva SCC | Other |
|---|---|---|---|---|---|---|
| CK20+ (Epith+) | 9/9(100%) | 6/6(100%) | 30/35(86%) | - | 3/4(75%) | - |
| CK20+ (Epith-) | - | 0/15(0%) | 1/13(8%) | 1/13(8%) | 0/3(43%) | 1/4(25%) |

FIGURE 1

EP 1 138 780 A1

**Description**

The invention relates to a method for the determination of cytokeratin 20 mRNA in tissue and body fluid samples of tumor patients and its use for identification of patients with the potential risk of tumor recurrence.

Many patients diagnosed with tumor-free margins suffer a recurrence of their disease. A popular explanation is that some of these cases recur due to the presence of disseminated cells, or micrometastases, that are currently undetectable by conventional methods (Pelkey, T.J., et al., Clinical Chemistry 42 (1996) 1369-1381). Reverse transcriptase-polymerase chain reaction (RT-PCR) is known as a method for the detection of circulating tumor cells. For such assays, cells are extracted from blood, bone marrow or lymph nodes by cytocentrifugation techniques and subsequent mRNA extraction. The mRNA is converted into cDNA through the use of specific primers and the enzyme reverse transcriptase, and the DNA is amplified is subsequently amplified by either nested or non-nested PCR. Usually, the PCR product is identified after gel electrophoresis or conventional detection methods such as detection of labels, or the like.

As markers for a micrometastasis, a lot of surface cell proteins are suggested. Common antigens which are useful as a basis for the specific primers are, for example, cytokeratins. Cytokeratins contribute to differentiation and function of the cytoskeleton of epithelial cells. Their determination in tissue or body fluid might be useful for tumor diagnosis.

Cytokeratin 20 (CK-20) is a major cellular protein of major enterocytes and globlet cells. CK-20 is a polypeptide with a molecular weight of 48,553 D and a member of the type 1-CK subfamily. The human gene of CK-20 is described by Moll, R., et al., Differentiation 53 (1993) 75-93. Its sequence is described in Swiss-Prot P35900.

Several attempts have been made to investigate whether detection of CK-20 and its mRNA expression is a marker for the detection of micrometastasis and minimal residual disease in patients with cancer of epithelial origin.

Soeth, E., et al. describe, in Int. J. Cancer 69 (1996) 278-282, a CK-20 nested RT-PCR examination in colorectal carcinoma cell lines and in bone marrow cells of patients with tumor stages I-IV (UICC Classification). Qualitative Control RT-PCR was performed for glyceraldehyde-3-phosphate dehydrogenase (GAPDH) for demonstrating the integrity of the detected RNA. Soeth et al. conclude that there is a relation between the expression of the epithelial marker CK-20 in the bone marrow and the UICC classified tumor stages.

Funaki, N.O., et al. describe, in British Journal of Cancer 77 (1996) 1327-1332, a three-step PCR assay for CK-20, using a simultaneously performed PCR without any template as a negative control, and tumor cell cDNA as a positive control. However, one of the drawbacks of Funaki's assay is, as pointed out in this publication, the possibility of contamination by normal epithelial cells which cannot be ruled out because of the lack of a definite marker to discriminate between carcinoma cells and non-cancerous cells. Funaki et al. assumed that the presence of CK-20 mRNA in the peripheral blood of advanced colorectal carcinoma patients seems to represent an indicator of possible recurrence in individual patients.

Buchumensky, V., et al. (J. Urology 160 (1998) 1971-1974) examined whether CK-20 expression can be used as a bladder tumor marker for transitional cell carcinoma in cells isolated from urine. As a method there is also used RT-PCR. Buchumensky et al. conclude, from their results, that there is no correlation between CK-20 and tumor grade and that there is also no correlation between CK-20 and tumor stage. Since RT-PCR is a sensitive method which is able to detect expression of cytokeratins in a small number of cells, Buchumensky et al. feel it is no wonder that CK-20 is not suitable for evaluation of grading and staging.

Bostick, P.J., et al. describe, in J. Clinical Oncology 16 (1998) 2632-2640, a CK-20 mRNA RT-PCR method for the detection of CK-20 in breast cancer cell lines and primary breast tumor cells. CK-20 could not be detected by Bostick et al. in lymph nodes of patients without cancer but in 75% of primary breast tumors and in 14% of sentinel lymph nodes (SN) of such patients.

Champelovier, P., et al. (Anticancer Research 19 (1999) 2073-2078) describe a method for the detection of CK-20 mRNA by nested PCR using primers within exons 2 and 3. The detection of the PCR products is carried out after ethidium bromide staining and electrophoresis. Negative controls were performed by nested PCR from non-tumor cells with the same primers. Champelovier found that CK-20 mRNA expression is not a sufficiently specific epithelial cell marker to allow its use for minimal residual disease detection for tumors of epithelial origin. Champelovier, in addition, found that the detection of CK-20 mRNA in blood samples using nested RT-PCR is not informative.

Weitz, J., et al. describe, in Clinical Cancer Research 5 (1999) 1830-1836, the detection of CK-20 mRNA by RT-PCR assays. They conclude that the detection of tumor cells in the apical lymph node by CK-20 RT-PCR has prognostic relevance. However, they concluded that the detection of tumor cells in surgically removed lymph nodes by CK-20 RT-PCR cannot be a prognostic marker per se.

Bustin, S.A., et al. describe, in British Journal of Cancer 79 (1999) 1813-1820, the detection of CK-20 in peripheral blood of colorectal cancer patients. It is concluded that there was no apparent difference in CK-20 mRNA transcription levels between controls and patients or between patients with different Dukes' stages. It is therefore concluded that CK-20 is no reliable marker for the detection of colon epithelial cells in peripheral blood. The reason is that CK-20 expression occurs, according to Bustin et al., too frequently in the peripheral blood of healthy volunteers.

Weber, T., et al. describe, in British Journal of Cancer 82 (2000) 157-160, a method for the detection of CK-20 mRNA by RT-PCR of samples of patients with thyroid carcinoma. They found that for thyroid carcinoma cells CK-20 mRNA seems to be a useful preoperative diagnosis tool of thyroid cancer.

All PCR assays for CK-20 described in the state of the art determine the absolute amount of PCR products using as a qualitative control, e.g., PCR products from non-epithelial cells or PCR results without templates (negative control) and epithelial tumor cells (positive control).

It was the object of the present invention to provide a method by which it is possible to quantitatively determine CK-20 in tissue samples and body fluid samples of patients and which enables a reproducible and quantitative determination of the tumor stage and of the potential for recurrence, minimal residual disease or micrometastases.

This object is accomplished by a method for the detection of cytokeratin 20 (CK-20) mRNA in a tissue or a body fluid sample of a patient characterized by

i) isolating RNA from said sample;

ii) preparing cDNA from said RNA by reverse transcription and dividing said cDNA into two compartments;

iii) performing polymerase chain reaction (PCR) with CK-20 specific primers in the first compartment;

iv) performing PCR with primers specific for a housekeeping gene in the second compartment;

v) calculating the relative ratio of mRNA of CK-20 to the housekeeping gene from the results of steps iii) and iv) as means for amount of the CK-20 mRNA in the sample.

It was surprisingly found that such an assay according to the invention enables for the first time the distinction of basal CK-20 mRNA levels from background CK-20 mRNA levels and establishes a reproducible link between the detection of CK-20 and metastatic potential. In addition, it was surprisingly found that CK-20 is a sensitive and specific marker of tumor recurrence and/or metastasis potential especially for colon carcinoma, breast cancer, head and neck cancer and melanoma.

A further object of the invention is the use of the method according to the invention for tumor staging. It was surprisingly found that the relative ratio of CK-20 to the housekeeping mRNA varies within a wide range between 1 and 100,000. Based on these findings, it is now possibe to identify patients having the potential of metastasis and recurrence also if no disseminated tumor cells can be detected in histology of lymph nodes.

Preferred ranges are approximately 1:10, 100 : 1000, 1000 : 10,000 and 10,000 : 100,000. In addition, it was found that the ratio is not only influenced by the tumor stage but also by the type of tumor. Whilst the ratio for colon carcinoma varies essentially between 10 and 100,000, the ratio for breast cancer varies between 1 and 10 (from lymph nodes, as specimens). However, this range too allows discrimination of tumor stages for breast cancer patients and the decision on a chemotherapy adjuvant to tumor excision, also if a negative histological result is found.

Lymph node or primary tumors sections are preferred as samples, however, it is also possible to use blood or bone marrow. The lymph node or primary tumour sections are either fresh samples or frozen, preferably snap-frozen in liquid nitrogen, and stored at about -80°C. Blood and bone marrow samples, upon collection, are stored and their cells lysed using preferably a guanidine hydrochloride-based solution with detergent. Additional steps to enrich specific cell fractions in blood, such as Ficoll-gradient separation, can also be used prior to cell lysis.

If samples such as blood or bone marrow are used, it is preferred to perform an enrichment of epithelial cells prior to mRNA extraction. Such an enrichment can be done by the use of epithelial specific binding such as immuno beads or high gradient + magnetic cell sorting (MACS) (Hardingham, I.E., et al., Int. J. Cancer 20 (2000) 8-13; Martin, V.M., et al., Exp. Hematology 26 (1998) 252-264).

For the extraction of mRNA, lymph node or primary tumor material is homogenized and their cells lysed using a guanidine hydrochloride-based solution with detergent, equivalent to that used for blood or bone marrow sample storage. From there, mRNA is isolated from all sample types by either precipitation or column affinity purification.

Reverse transcription of mRNA into cDNA is conducted according to commonly recommended protocols. It is preferred that random hexamers are used to prime cDNA synthesis as opposed to oligo dT or specific sequence primers. Hexamers primers are more robust to mRNA quality than oligodT primers and produce cDNA that can be used in multiple PCR reactions unlike specific priming.

PCR amplification of cDNA, detection and quantification is performed preferably on a quantitative real-time PCR machine such as the LightCycler® Instrument (Roche Diagnostics GmbH, DE). Preferably, cDNA is amplified for 50 cycles to allow the lowest copy numbers to be detected.

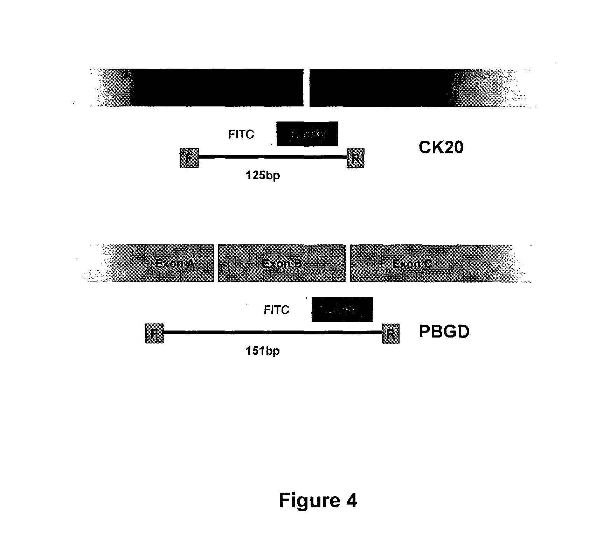

It is preferred that primers and hybridisation probes for CK-20 and especially for the housekeeping gene are positioned to solely prime cDNA derived from mRNA and exclude amplification of DNA, pseudogene and/or splice-site variant amplification. Firstly, regions of the gene not subject to pseudogene and splice-site variant amplification must be located as the primers should be placed outside said regions. Secondly, the primers should be placed in different exons - preferably with a large intron region in between said exons, or over exon-intron boundaries (see Fig. 4). In this manner, amplification of DNA instead of mRNA will be excluded, particularly when short extension times are used. In addition, one of the hybridisation probes is preferably placed over an intron-exon boundary, thereby allowing detection (preferably fluorescence) only when bound to the mRNA sequence.

Another preference is the amplification of short fragments between 100-200 base pairs. Compared to larger fragments, amplification of short fragments allows shorter run times, is more sensitive at low copy numbers and is more robust for amplification of degraded mRNA samples.

Detection and quantification of CK20 is conducted by calculating the relative ratio between the quantity of CK20 and that of a housekeeping gene. By "housekeeping genes" in the sense of the invention are meant genes whose mRNA amount is, after expression, essentially independent of external influences or of the cell type (especially, independently of whether the cell is a tumor cell or a non-tumor cell). Suitable housekeeping genes in the sense of the invention are those whose mRNA is the sole source of amplified product. Many housekeeping genes have pseudogenes or splice-site variants. PCR primer positioning as described above is an important criterion to avoid non-specific amplification.

Genes commonly used as housekeeping genes include glyceraldehyde-3-phosphate dehydrogenase (GAPDH), beta-actin and beta-2-microglobin. Preferred housekeeping genes are porphobilinogen deaminase (PBGD) and glyceraldehyde-6-phosphate dehydrogenase (G6PDH) due to their reduced problems with non-specificity and lower mRNA expression levels compared to other housekeeping genes.

The amount of CK20 is given as the ratio of CK20 to housekeeping mRNA preferably relative to that from a calibrator sample and is calculated in multiple steps. Firstly, the mRNA copy numbers of each are calculated from the efficiency of the PCR reaction raised to the power of the crossing point (beginning of the exponential phase of the PCR reaction), that is $\text{Efficiency}^{\text{Crossing Point}}$. The efficiency of the PCR reaction, normally around 2, is determined empirically prior to these analysis by determining the rate of reaction from multiple sample dilutions. Secondly, the ratio of CK20 to housekeeping mRNA copies is calculated for the sample by division. In addition, preferably this ratio for the sample is divided by the ratio from a calibrator that is run in parallel. The calibrator is a mRNA sample containing a standardised CK20 to housekeeping gene ratio and is analysed with each PCR to normalise inter-PCR variations. As a source for said mRNA every reproducible available cell line like ATCC TB-38 can be used.

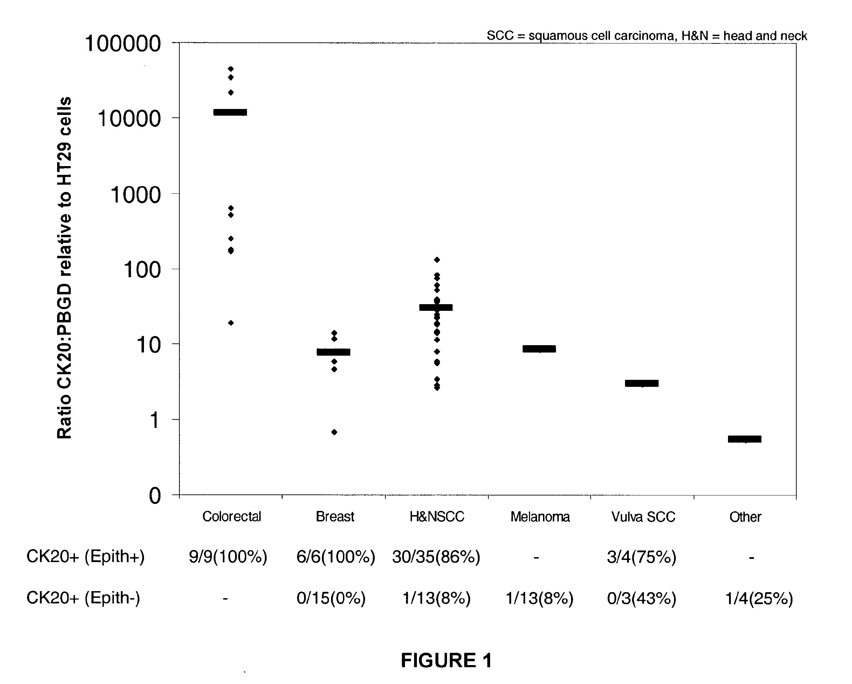

A further object of the invention is the use of a chemotherapy adjuvant to tumor excision for patients with histologically tumor negative lymph nodes but with a ratio CK-20 : housekeeping gene of 1 and greater in the CK-20 mRNA assay according to the invention, preferably detected in lymph node samples. According to Fig. 1 it is possible with the assay according to the invention to identify from the group of patients with histologically negative lymph nodes a subgroup of patients showing considerable CK-20 mRNA from, preferably, lymph node specimens. This leads to the conclusion that disseminated tumor cells are located in the lymph nodes of such patients, and that the amount of these tumor cells is, however, extremely low so that they cannot be detected by conventional histological methods. The highly sensitive assay according to the invention is capable, however, of detecting such tumor cells, especially in lymph nodes. This leads to the conclusion that such patients should be treated with an adjuvant chemotherapy to avoid recurrence.

As chemotherapy there is applied that chemotherapy which would be applied to a patient in the case of a histologically positive lymph node.

Such histological methods are described, for example, by Goldstein, N.S., et al., Am. J. Clin. Pathol. 111 (1999) 51-58; Kanamura, H., et al., Int. J. Urol. 4 (1997) 451-455; Natsugoe, S., et al., Japan J. Surg. 21 (1991) 528-532; Vander Valk, P., et al., Am. J. Surg. Pathol. 11 (1987) 866-882.

A further object of the invention is a method for the treatment of a tumor patient, adjuvant to tumor excision, by chemotherapy, showing histologically tumor negative lymph nodes but with a ratio of CK-20: housekeeping gene of 1 and greater in the CK-20 mRNA assay according to the invention, preferably detected in lymph nodes samples of said patient.

A further object of the invention is the use of a chemotherapeutic agent for the manufacture of a pharmaceutical agent containing said chemotherapeutic agent for the treatment of a tumor patient, adjuvant to tumor excision, said patient showing histologically tumor negative lymph nodes but with a ratio of CK-20 : housekeeping gene of 1 and greater in the CK-20 mRNA assay according to the invention, preferably detected in lymph nodes samples.

As chemotherapeutic agents all chemotherapeutics are useful with which patients are treated after tumor excision and a histologically tumor positive lymph nodes result. Such chemotherapeutic agents are widely known in the state of the art. The type of chemotherapeutic agent depends specifically on the type of tumor. Chemotherapeutic agents for bladder tumors are described in Sternberg, C.N., et al., BJU Int. 85 (2000) 599-610). Adjuvant chemotherapy for colon and rectal cancer is described in Fuchs, C.S., et al., Semin. Radiat. Oncol. 5 (1995) 472-487. Chemotherapy for breast cancer is described in Recht, A., et al., Semin. Radiat. Oncol. 2 (1992) 107-115; Sapunar, F., Ann. Med. 32 (2000) 43-50; and Lebwohl, D.E., Ann. Onc. 10, Suppl. 6 (1999) 139-146. Chemotherapy for melanoma is described in Green, R.J., et al., Hematol. Oncol. Clin. North Am. 12 (1998) 863-875; and Atkins, M.B., Curr. Opin. Oncol. 9 (1997) 205-213). Chemotherapy for vulva carcinoma is described in Coh, W.J., et al., Int. J. Radiat. Oncol. Biol. Phys. 26 (1993) 809-816. Chemotherapy of head and neck cancer is described in Forastiere, A.A., Acta Otorhinolaryngol. Belg.

53 (1999) 277-280; and de Mulder, P.H., Anticancer Drugs 10, Suppl. 1 (1999) S33-S37.

The following examples, references, sequence listing and figures are provided to aid the understanding of the present invention, the true scope of which is set forth in the appended claims. It is understood that modifications can be made in the procedures set forth without departing from the spirit of the invention.

## Description of the Figures

**Figure 1**   Incidence and quantity of CK-20 in lymph nodes from colorectal and non-colorectal tumors.
(Epith+/-): epithelial cells found/not found in histology; SCC: squamous cell carcinoma; H&N: head and neck cancer.

**Figure 2**   Incidence and quantity of CK-20 between epithelial cell negative and positive lymph nodes (detected in histology) from non-colorectal tumors. Negative/Positive: negative or positive according to epithelial cell invasion detected by histology.

**Figure 3**   Incidence and quantity of CK-20 between tumors and lymph nodes from colorectal and breast cancer patients.
CrC: colorectal carcinoma; BrC: breast cancer.

**Figure 4**   Placement of CK-20 and PBGD primers (F and R) and hyb probes (FITC and LC640) across exon/intron boundaries.

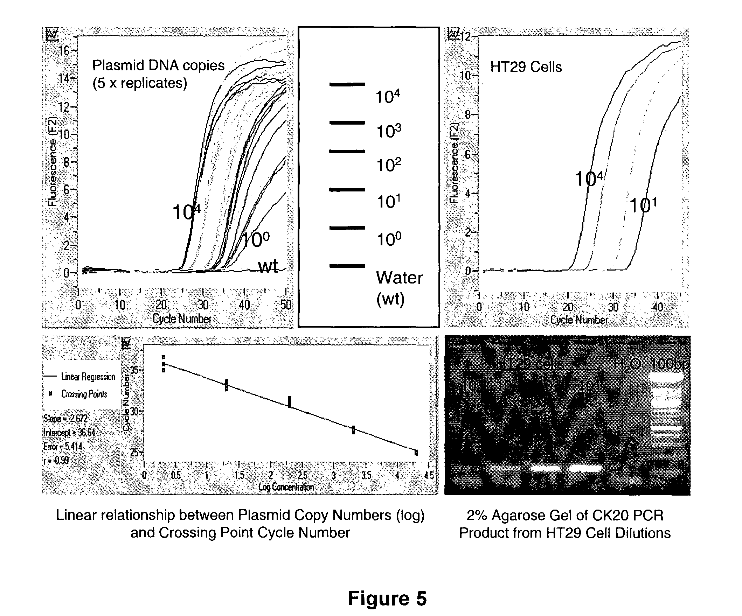

**Figure 5**   Light Cycler® detection and quantification of CK-20 mRNA: Sensitivity, specificity and reproducibility.

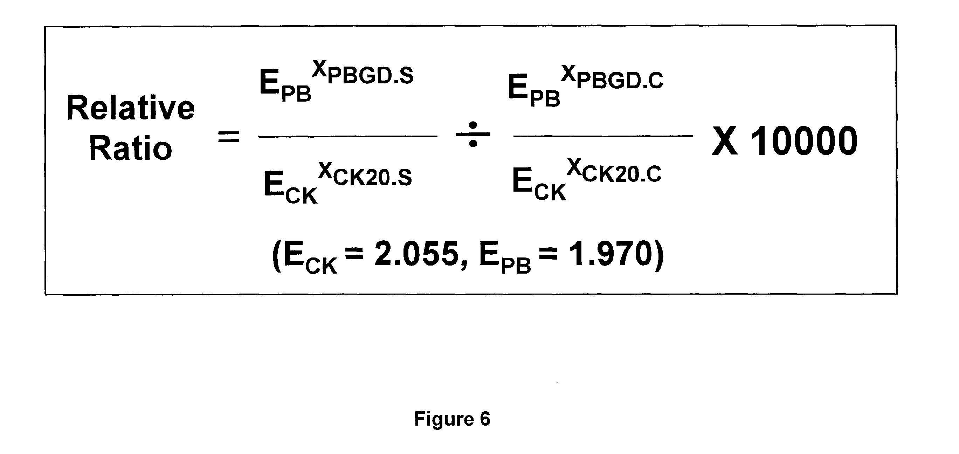

**Figure 6**   Calculation of the relative ratio of CK-20 mRNA to PBGD mRNA.
S: sample; C: calibrator.

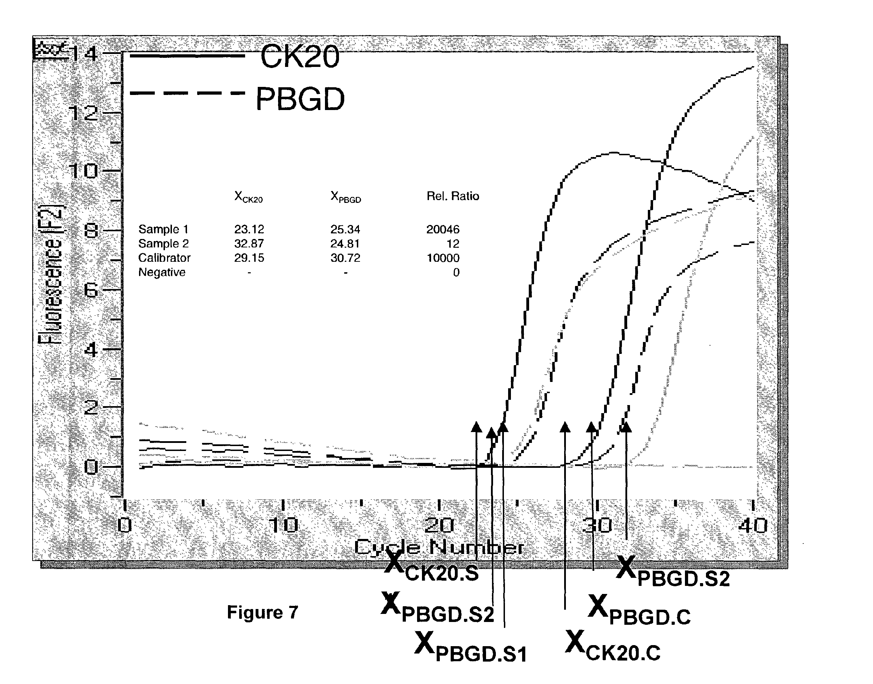

**Figure 7**   Calculation of X values for CK-20 and PBGD.
S1/S2: sample 1 or 2; C: calibrator; E: efficiency, see Example 3.

## Example 1

**Sample preparation and mRNA extraction from primary tumors and lymph nodes**

Lymph nodes and primary tumours were snap-frozen in liquid nitrogen and stored at - 80°C. Approximately 50-150mg frozen tissue was homogenised and sample RNA extracted using the TriPure Reagent® (clear, red, monophasic solution of phenol and guanidine thiocyanate, pH 4, Roche Diagnostics GmbH, DE). RNA from the HT29 colon carcinoma cell line (ATCC #TB-38) was extracted using the HighPure® Isolation Kit (Roche Diagnostics GmbH, DE). The kit is designed for the preparation of total RNA mini-preparations (free of contaminating DNA). Samples are lysed and homogenized in the presence of chaotropic salt and then applied to a spin filter tube. Nucleic acids bind specifically to the surface of the filter. Co-purified DNA is ultimately digested with DNAse I. The bound RNA is purified from salts, proteins, digested DNA and other impurities, by washing steps followed by an elution. Calibrator RNA consisted of 1ng/μl HT29 RNA.

## Example 2

**cDNA preparation**

mRNA is reverse transcribed into cDNA in a reaction containing 20 units AMV reverse transriptase (RT), 1xAMV buffer (50mM Tris, pH 8.5, 30mM KCl, 1mM DTT, 8mM MgCl$_2$, 1mM deoxynucleotide triphosphates and 0.0625A$_{260}$ random hexamers. The reaction is performed by incubation at 25°C for 10 minutes, 42°C for 30 minutes and 94°C for 5 minutes. Calibrator mRNA is included for each RT run.

### Example 3

**Carrying out of the PCR with results for different tumors, short explanation of Figs. 1-3**

PCR detection and quantification of CK20 is performed on the LightCycler® Instrument. Two PCRs are performed for each sample, one for CK20 and the other for PBGD. The PCR is performed in a 20µl reaction volume consisting of 2µl cDNA, 2µl of 10x DNA Hybridisation Probes Buffer, 2µl of 10x Detection Mix and 14µl water. Each PCR consists of an initial 1 minute denaturation at 95°C, then 50 cycles of 95°C for 0 seconds, 60°C for 10 seconds and 72°C for 5 seconds before cooling at 40°C for 30 seconds. During the 10 seconds at 60°C, the flourescence signal is acquired. At the end of the run, flourescence vs cycle number is plotted and the crossing point (start of PCR exponential phase) calculated using the LightCycler® software.

The final result is a ratio of CK20 to PBGD relative to calibrator RNA and is calculated according to the following equation (Fig. 1)

$$\text{Ratio} = (E_{PBGD}^{X\text{-}PBGD.Sample}/E_{CK20}^{X\text{-}CK20.Sample})/(E_{PBGD}^{X\text{-}PBGD.Calibrator}/E_{CK20}^{X\text{-}CK20.Calibrator})$$

where X = crossing point

The efficiency is pre-determined at 2.085 for CK20 and 1.097 for PBGD. This was evaluated using serial dilutions of multiple samples to determine the reaction rate according to the equation

$$\text{Efficiency} = \log^{-1/slope}$$

where slope is calculated from the linear relationship between crossing point and sample concentration.

Using this method, distinct differences in CK20 mRNA levels were detected between lymph nodes from colorectal and non-colorectal tumours including breast carcinoma, head and neck squamous cell carcinoma, melanoma and vulval squamous cell carcinoma (Fig. 1). The frequent and reliable detection of CK20 in the non-colorectal cancers was a surprising finding.

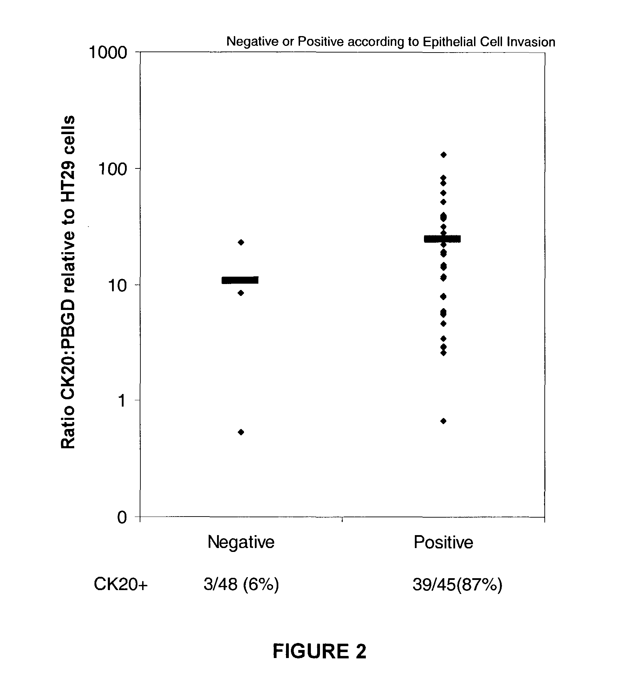

In lymph nodes, CK20 mRNA detection associated with the presence of epithelial invasion as determined histologically (Fig. 2). Some 39/45 (87%) of lymph nodes with epithelial invasion were CK20 positive whilst 3/48 (6%) epithelial negative cells were CK20 positive.

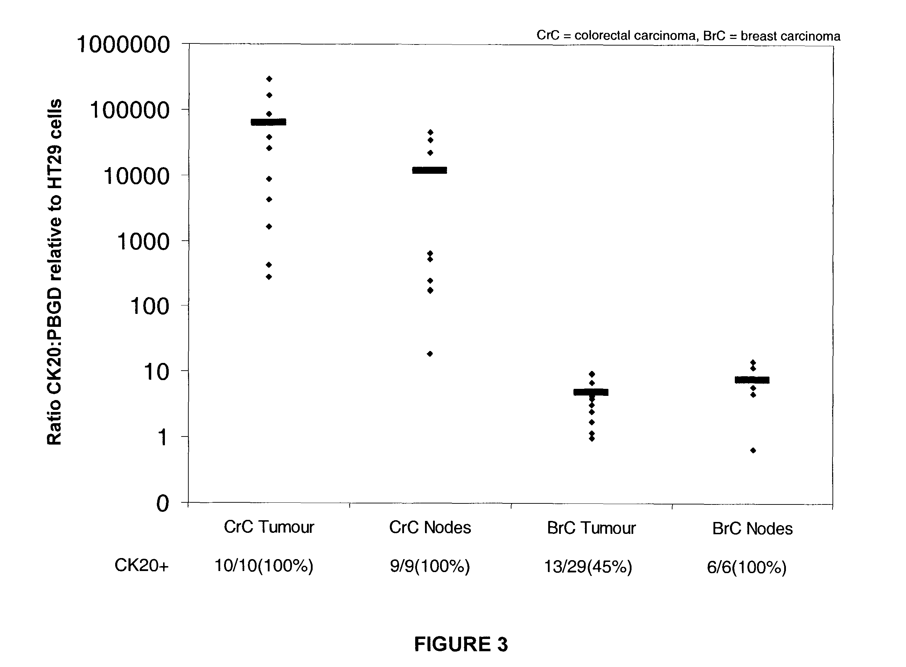

CK20 levels were found to be relatively consistent between primary tumour and lymph nodes in that the levels were both high for colorectal cancer and both low for breast cancer (Fig. 3).

### List of References

Atkins, M.B., Curr. Opin. Oncol. 9 (1997) 205-213
Bostick, P.J., et al., J. Clinical Oncology 16 (1998) 2632-2640
Buchumensky, V., et al., J. Urology 160 (1998) 1971-1974
Bustin, S.A., et al., British Journal of Cancer 79 (1999) 1813-1820
Champelovier, P., et al., Anticancer Research 19 (1999) 2073-2078
Coh, W.J., et al., Int. J. Radiat. Oncol. Biol. Phys. 26 (1993) 809-816
de Mulder, P.H., Anticancer Drugs 10, Suppl. 1 (1999) S33-S37
Forastiere, A.A., Acta Otorhinolaryngol. Belg. 53 (1999) 277-280
Fuchs, C.S., et al., Semin. Radiat. Oncol. 5 (1995) 472-487
Funaki, N.O., et al., British Journal of Cancer 77 (1996) 1327-1332
Goldstein, N.S., et al., Am. J. Clin. Pathol. 111 (1999) 51-58
Green, R.J., et al., Hematol. Oncol. Clin. North Am. 12 (1998) 863-875
Hardingham, I.E., et al., Int. J. Cancer 20 (2000) 8-13
Kanamura, H., et al., Int. J. Urol. 4 (1997) 451-455
Lebwohl, D.E., Ann. Onc. 10, Suppl. 6 (1999) 139-146
Martin, V.M., et al., Exp. Hematology 26 (1998) 252-264
Moll, R., et al., Differentiation 53 (1993) 75-93
Natsugoe, S., et al., Japan J. Surg. 21 (1991) 528-532
Pelkey, T.J., et al., Clinical Chemistry 42 (1996) 1369-1381

Recht, A., et al., Semin. Radiat. Oncol. 2 (1992) 107-115
Sapunar, F., Ann. Med. 32 (2000) 43-50
Soeth, E., et al., Int. J. Cancer 69 (1996) 278-282
Sternberg, C.N., et al., BJU Int. 85 (2000) 599-610
Vander Valk, P., et al., Am. J. Surg. Pathol. 11 (1987) 866-882
Weber, T., et al., British Journal of Cancer 82 (2000) 157-160
Weitz, J., et al., Clinical Cancer Research 5 (1999) 1830-1836

**Claims**

1. Method for the detection of cytokeratin 20 (CK-20) mRNA in a tissue or body fluid sample of a patient, **characterized by**

   i) isolating RNA from said sample;
   ii) preparing cDNA from said RNA by reverse transcription and dividing said cDNA into two compartiments;
   iii) performing polymerase chain reaction (PCR) with CK-20 specific primers in the first compartiment;
   iv) performing PCR with primers specific for a housekeeping gene in the second compartiment;
   v) calculating the relative ratio of mRNA of CK-20 to the housekeeping gene from the results of steps iii) and iv) as means for amount of the CK-20 mRNA in the sample.

2. The method according to claim 1, **characterized in that** as a housekeeping gene porphobilinogen deaminase (PBGD) or glyceraldehyde-6-phosphate dehydrogenase (G6PDH) is used.

FIGURE 1

Ratio CK20:PBGD relative to HT29 cells

Negative or Positive according to Epithelial Cell Invasion

CK20+    3/48 (6%) Negative    39/45(87%) Positive

FIGURE 2

FIGURE 3

Figure 4

**Figure 5**

Plasmid DNA copies
(5 × replicates)

$10^4$

$10^0$

Wt

Water (wt)

$10^0$

$10^1$

$10^2$

$10^3$

$10^4$

HT29 Cells

$10^4$

$10^1$

Linear Regression

Crossing Points

Slope = 2.672
Intercept = 36.64
Error = 5.414
$r = 0.99$

Linear relationship between Plasmid Copy Numbers (log)
and Crossing Point Cycle Number

2% Agarose Gel of CK20 PCR
Product from HT29 Cell Dilutions

$$\text{Relative Ratio} = \frac{E_{PB}^{X_{PBGD.S}}}{E_{CK}^{X_{CK20.S}}} \div \frac{E_{PB}^{X_{PBGD.C}}}{E_{CK}^{X_{CK20.C}}} \times 10000$$

$$(E_{CK} = 2.055,\ E_{PB} = 1.970)$$

Figure 6

Figure 7

EP 1 138 780 A1

**European Patent Office**

**EUROPEAN SEARCH REPORT**

Application Number

EP 00 10 7036

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | FUNAKI N O ET AL.: "Perioperative quantitative analysis of cytokeratin 20 mRNA in peripheral venous blood of patients with colorectal adenocarcinoma " ONCOLOGY REPORTS, vol. 7, 2000, pages 271-276, XP000949330 * the whole document * | 1,2 | C12Q1/68 |
| D,X | BUSTIN S A ET AL.: "Detection of cytokeratins 19/20 and guanylyl cyclase C in peripheral blood of colorectal cancer patients" BRITISH JOURNAL OF CANCER, XP000949369 * the whole document * | 1,2 | |
| X | SOONG R AND TABITI K: "Detection of colorectal micrometastasis by quantitative RT-PCR of cytokeratin 20 mRNA" PROCEEDINGS OF THE AMERICAN ASSOCIATION FOR CANCER RESEARCH, vol. 41, 2000, page 391 XP002149389 * abstract * | 1 | TECHNICAL FIELDS SEARCHED (Int.Cl.7) C12Q |
| D,X | FUNAKI N O ET AL.: "Cytokeratin 20 mRNA in peripheral venous blood of colorectal carcinoma patients" BRITISH JOURNAL OF CANCER, vol. 77, no. 8, 1998, pages 1327-1332, XP000949368 * the whole document * | 1 | |

-/--

The present search report has been drawn up for all claims

| Place of search | Date of completion of the search | Examiner |
|---|---|---|
| THE HAGUE | 5 October 2000 | Knehr, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

15

European Patent Office

## EUROPEAN SEARCH REPORT

Application Number

EP 00 10 7036

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | KNEHR ET AL: "CELLULAR EXPRESSION OF HUMAN CENTROMERE PROTEIN C DEMONSTRATES A CYCLIC BEHAVIOR WITH HIGHEST ABUNDANCE IN THE G1 PHASE" PROCEEDINGS OF THE NATIONAL ACADEMY OF SCIENCES OF USA,NATIONAL ACADEMY OF SCIENCE. WASHINGTON,US, vol. 93, September 1996 (1996-09), pages 10234-10239, XP002100369 ISSN: 0027-8424 * abstract * * page 10235, column 2, paragraph 2 - paragraph 3 * * page 10237, column 2, paragraph 2; figure 4 * | 1,2 | |
| Y | WO 94 23023 A (UNIV ROCHESTER) 13 October 1994 (1994-10-13) * the whole document * | 1,2 | |
| D,A | SOETH E: "The detection of disseminated tumor cells in bone marrow from colorectal-cancer patients by a cytokeratin-20-specific nested reverse-transcriptase-polymerase-chain reaction is related to the stage of disease" INTERNATIONAL JOURNAL OF CANCER, vol. 69, 1996, pages 278-282, XP000949379 * the whole document * ---<br>-/-- | | TECHNICAL FIELDS SEARCHED (Int.Cl.7) |

The present search report has been drawn up for all claims

| Place of search | Date of completion of the search | Examiner |
|---|---|---|
| THE HAGUE | 5 October 2000 | Knehr, M |

**European Patent Office**

## EUROPEAN SEARCH REPORT

Application Number

EP 00 10 7036

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | WEBER T ET AL.: "Expression of cytokeratin 20 in thyroid carcinomas and peripheral blood detected by reverse transcription polymerase chain reaction" BRITISH JOURNAL OF CANCER, vol. 82, no. 1, 2000, pages 157-160, XP000949370 * the whole document * | | |
| D,A | CHAMPELOVIER P ET AL.: "CK20 gene expression: Technical limits for the detection of circulating tumor cells" ANTICANCER RESEARCH, vol. 19, 1999, pages 2073-2078, XP000949390 * the whole document * | | |

TECHNICAL FIELDS
SEARCHED      (Int.Cl.7)

The present search report has been drawn up for all claims

| Place of search | Date of completion of the search | Examiner |
|---|---|---|
| THE HAGUE | 5 October 2000 | Knehr, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 138 780 A1**

**ANNEX TO THE EUROPEAN SEARCH REPORT
ON EUROPEAN PATENT APPLICATION NO.**                    EP 00 10 7036

This annex lists the patent family members relating to the patent documents cited in the above–mentioned European search report.
The members are as contained in the European Patent Office EDP file on
The European Patent Office is in no way liable for these particulars which are merely given for the purpose of information.

05-10-2000

| Patent document cited in search report | Publication date | Patent family member(s) | Publication date |
|---|---|---|---|
| WO 9423023 A | 13-10-1994 | US 5643765 A<br>US 5639606 A<br>AU 6493194 A<br>JP 7132100 A<br>US 5876978 A | 01-07-1997<br>17-06-1997<br>24-10-1994<br>23-05-1995<br>02-03-1999 |